# EUROPEAN PATENT APPLICATION

(11) **EP 3 854 226 A1**
(43) Date of publication of application: **28.07.2021**
(21) Application number: 21153111.6
(22) Date of filing: 22.01.2021
(51) Int. Cl.: A23L 19/00, A23L 33/105, A61K 8/9789, A61K 36/63

(54) **METHOD FOR FRACTIONATING OLIVE POMACE, ITS PRODUCTS AND USES THEREOF**

(30) Priority: 22.01.2020 PT 2020116072
(71) Applicant: Universidade Católica Portuguesa - UCP, 4169-005 Porto (PT); Associação BLC3 - Campus de Tecnologia e Inovação, 3405-155 Oliveira do Hospital (PT); Universidade do Minho, 4704-553 Braga (PT)
(72) Inventor: ESTEVEZ PINTADO, MARIA MANUELA, 4169-005 PORTO (PT); BRAGANÇA RIBEIRO, TÂNIA ISABEL, 4169-00 PORTO (PT); OLIVEIRA, ANA LÚCIA, 4169-005 PORTO (PT); VICENTE, ANTÓNIO, 4704-553 BRAGA (PT); NUNES, JOÃO, 3405-155 LAGARES OHP (PT)
(74) Representative: Patentree

(57) **Abstract**

The present disclosure relates to the field of agriculture, namely to the olive oil industry. It provides a procedure to obtain value-added products from olive pomace with the goal of zero waste while maximizing the value of the products.

The present disclosure also relates to the field of solid biofuels, cosmetics, medicine and food industry. The products obtained by the different methods herein disclosed can be used in a broad range of fields. The products obtained may be used as solid biofuel, cosmetic exfoliation material and food ingredients.

The present disclosure uses environmentally friendly methodologies of fractionation and blanching, belonging therefore also to the field of ecology.

## Description

### TECHNICAL FIELD

The present disclosure relates to the field of agriculture, namely to the olive oil industry. It provides a procedure to obtain value-added products from olive pomace with the goal of zero waste while maximizing the value of the products.

The present disclosure also relates to the field of solid biofuels, cosmetics, medicine and food industry. The products obtained by the different methods herein disclosed can be used in a broad range of fields. The products obtained may be used as solid biofuel, cosmetic exfoliation material and food ingredients.

The present disclosure uses environmentally friendly methodologies of fractionation and blanching, belonging therefore also to the field of ecology.

### BACKGROUND ART

Nowadays, olive oil industry is turning to one of the larger agro-food industries in the European Union, but also one of the most polluting industries (Filotheou et al., 2015). From 100 kg of olives, 20-30 kg of olive oil and 60-80 kg of olive pomace are produced. Olive pomace is the most representative by-product of olive oil extraction process as result of the growing implementation of the eco-friendlier two-phase extraction method (no water is added) as a substitute for the three-phase method (Nunes, Pimentel, Costa, Alves, & Oliveira, 2016). Although the extensive reduction of liquid residue, the two-phase process leads to a slight increase in solid wastes (60% higher) (Rincón, Fernando, G., & Borja, 2012).

Olive pomace is a humid semi-solid, which is phytotoxic, non-biodegradable and challenging to treat because of its high water and organic compounds content (Esteve, Marina, & Garcia, 2015; Lama-Muñoz, Rodríguez-Gutiérrez, Rubio-Senent, & Fernández-Bolaños, 2012).

Commonly, olive pomace is used directly as solid biofuel in direct combustion technology after an oil extraction and drying process (Muíño et al., 2017; Rubio-Senent, Rodríguez-Gutiérrez, Lama-Muñoz, & Fernández-Bolaños, 2015). This direct combustion of olive pomace exhibited several disadvantages: the impurities such as the olive pulp and the olive stone fines particles are negative compounds related to uncontrolled combustion with emissions, corrosions and slagging (Mata Sánchez et al., 2015). Besides, the direct combustion also represents a wastage of bioactive compounds to generate a final product (heat and/or electricity) with comparatively lower economic value.

Several works exploited the phenolic compounds (only a small fraction of olive pomace), leaving a high majority of olive pomace left without valorisation. Olive pomace presents a significant amount of other compounds with value for food formulation, cosmetic and energy, which have been neglected in most of the approaches developed until now.

The development of functional foods with health-promoting benefits and fortified foods has increased in the past decade (Helal & Tagliazucchi, 2018). Consumers demand for natural functional and fortified food products is still increasing, with particular concern about health benefits (Nocella & Kennedy, 2012) and nutritional aspect of the food (Tomic et al., 2017).

For example, WO2014027054A2 describes a process to obtain a solid biofuel in the form of pellets composed by residues of fruit or berries (peel, pulp, endocarp, stones) and organic adhesive, and comprising less than 15% of water, preferably less than 10% of water. The solid biofuel is preferably based on olive residues and comprises more than 40% by weight of ground endocarps with a particle size of between 3 and 9 mm.

Another example, WO2018109600A1 provides for an extract from olive pomace that comprises a mixture of oil-soluble and water-soluble compounds including hydroxytyrosol, tyrosol, oleuropein, α-tocopherol, and squalene. The invention further provides for a method of producing the extract, and formulations comprising the extract. Other patents have been described to obtain olive polyphenols concentrates, but the final solid only have been explored as solid substrate in patent WO2017212450A1. The valorization of whole biomass and components of olive pomace has been neglected.

WO2013030426 discloses a method for obtaining olive oil and at least one multifunctional ingredient from olives. This method is characterized in that it comprises the following steps: (a) obtaining olive pulp, (b) dehydrating the olive pulp to produce a dehydrated olive pulp, (c) grinding the dehydrated olive pulp to produce a dry dehydrated olive powder, and (d) obtaining olive oil and at least one multifunctional ingredient from dry dehydrated olive powder.

Olive pomace is a mixture of water, residual skin, pulp and pieces of the crushed stone. The stones ≥ 1 mm in size exhibit a potential application for thermal power generation without risk of uncontrolled combustions (Mata Sánchez et al., 2015). The small stones/peel can be incorporated in cosmetic formulations as an exfoliating agent and also may act as functional ingredients (Rodrigues, Pimentel, & Oliveira, 2015). On the other hand, the liquid fraction is a promising source of antioxidant compounds, mainly the phenolic compounds hydroxytyrosol and derivatives (Mirabella, Castellani, & Sala, 2014). The pulp fraction contains a considerable amount of fiber and bound phenolic compounds (Alu'datt, Rababah, Ereifej, & Alli, 2013), which may play a substantial role in gut health (Tabernero & Gómez de Cedrón, 2017). Several solutions for olive pomace management have been proposed, but the strategies proposed do not appear to answer completely the problematic.

There is therefore the need of developing a valorization process of whole olive pomace components to reach the "zero waste" goal/approach while maximizing the value of all waste producing value-added products with several applications, namely as solid biofuel, cosmetics, nutraceuticals and food ingredients.

### BRIEF DESCRIPTION OF DRAWINGS

The figures provide preferred embodiments for illustrating the description and should not be seen as limiting the scope of invention.
**Figure 1****:** Proposed integrated process for olive pomace valorisation to achieve the zero waste goal while maximizing the product: value added products.
**Figure 2****:** Concentrations of short chain fatty acids produced during fermentation of liquid-enriched fraction from olive pomace (LOPP) and controls (Negative - C-; Positive - FOS).
**Figure 3****:** Time inhibition curves drawn at different LOPP concentrations (1, 2 e 3%). (A) MSSA; (B) *Listeria monocytogenes;* (C) *Bacillus cereus;* (D) *Escherichia coli;* (E) *Yersinia enterocolitica;* (F) *Salmonella enteritidis.*

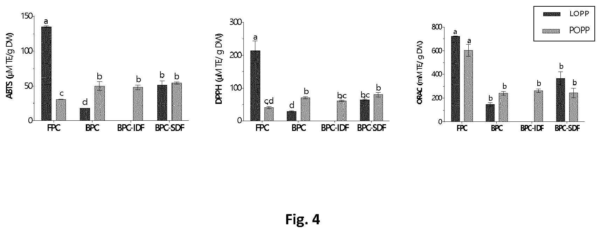

**Figure 4****:** Content and antioxidant activity using 2,2'-azino-bis(3-ethylbenzothiazoline-6-sulphonic acid (ABTS), 2,2-diphenyl-1-picrylhydrazyl (DPPH) and Oxygen Radical Absorbance Capacity (ORAC) methods of free and bounded phenolic compounds of olive pomace powders and its fiber fractions. FPC - Free phenolic compounds extract; BPC - Bound phenolic compounds extract; IDF - insoluble dietary fiber; SDF - Soluble dietary fiber. Results are the means of three determinations ± standard deviation. Different letters in the same column are significantly different as determined by ANOVA (p< 0.05).
**Figure 5****:** Concentrations of short chain fatty acids produced during fermentation of pulp-enriched fraction from olive pomace (POPP) and controls (Negative C-; Positive - FOS).
**Figure 6****:** Time inhibition curves drawn at different POPP concentrations (1, 2 e 3%). (A) MSSA; (B) Listeria monocytogenes; (C) Bacillus cereus; (D) Escherichia coli; (E) Yersinia enterocolitica; (F) Salmonella enteritidis.

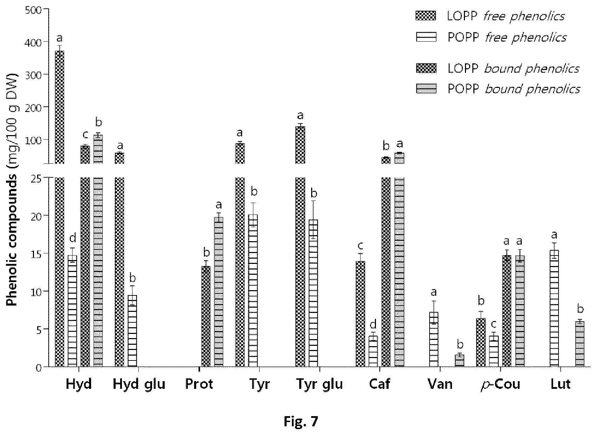

**Figure 7****:** Polyphenolic profile (mg/100 g sample dry weight) of olive pomace powders. Results are the means of three determinations ± standard deviation. Hyd - hydroxytyrosol; Hyd glu - Hydroxytyrosol glucoside; Prot - Protocatechuic acid; Tyr - Tyrosol; Tyr glu - Tyrosol glucoside; Caf - Caffeic acid; Van - Vannilin; *p* - Cou - *p* - Coumaric acid; Lut - Luteolin. Different letters in the same column are significantly different as determined by the t-Student test (p< 0.05).
**Figure 8****:** Metabolism of Caco-2 intestinal cells upon the presence of olive pomace powder at concentrations of 2, 1, 0.5, 0.25 and 0.125 % (w/v).

### GENERAL DISCLOSURE OF THE INVENTION

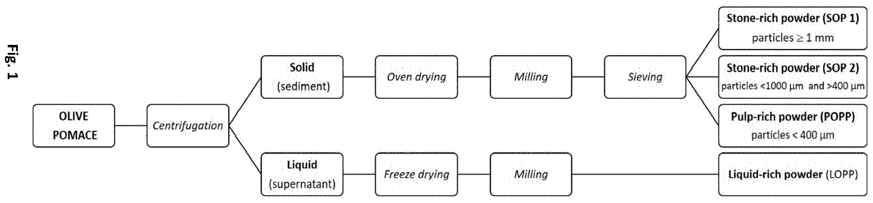

In this context, the present disclosure proposes a wet and dry fractionation strategy to achieve a maximal valorisation of all olive pomace components to reach the "zero waste" goal. The wet and dry fractionation valorisation scheme allowed the production of liquid-rich (LOPP), pulp-rich (POPP) and two stone-rich (SOP 1 and SOP 2) powders without any consumption of water nor chemicals and lower energy consumption in drying process **(****Fig.1****).**

In a second aspect of the present disclosure there is provided a method of producing powders rich in soluble fiber from pulp-rich fraction using H₂O₂.

The present disclosure comes as a response to the treatment and valorisation of all components of the high amount of waste obtained from olive oil industries, the olive pomace.

The present disclosure discloses a fractionation process to explore olive pomace as raw material to produce stone-rich (SOP 1 and SOP2), liquid-rich and pulp-rich powders as well as their respective applications and biological activities.

In a particular aspect of the present invention, the products obtained from stone rich fraction are added-value products which are used in a prioritized process, that use stones/peels (size <1000 µm and >400 µm, SOP2) as exfoliating agent for cosmetic products and, after this, the other fraction are used with a solid biofuel in the case of size particles ≥ 1 mm (SOP 1).

In an embodiment, the solid biofuel obtained from SOP 1 exhibits better characteristics than crude olive pomace, due to its composition free of oil, low moisture, uniform size (free of pulp and fine olive stone particles) and high density (Mata Sánchez et al., 2015). Moreover, the low nitrogen and sulphur percentage of stones minimise the NOₓ and SO₂ emissions associated with the occurrence of the acid rains and the destruction of the ozone layer (Rodriguez et al., 2008).

In a further embodiment, the small stones/peel (SOP 2) are also applied in cosmetic formulations as a functional ingredient, by improving physical and structural properties of hydration, oil holding capacity, emulsion and oxidative stability, viscosity, texture, sensory characteristics, and shelf life of cosmetic products.

In another embodiment, the LOPP achieved is rich in bioactive compounds and minerals that can be used as food additives to enhance the nutritional composition, but also as flavour enhancers and as preservatives to avoid food spoilage. Furthermore, the liquid-rich powder can be used to produce nutraceuticals, cosmetics or food supplements, owing to its richness in potassium and in olive oil polyphenols (hydroxytyrosol and derivatives).

In an embodiment, the POPP achieved is a powder with a high level of dietary fiber (principally insoluble dietary fiber) and a considerable amount of hydroxytyrosol and derivatives. The present powder is a source of antioxidant dietary fiber which exhibits the technological/health benefits of antioxidants and fiber simultaneously. Besides that, the POPP is a higher source of oleic acid. The high fiber and oleic acid content make this powder interesting to be used in human nutrition for its beneficial effects on the reduction of intestinal constipation and reduction of cholesterol and triglycerides, respectively.

In an embodiment, besides the direct use of the POPP, this powder is explored to obtain a blanched powder (BP) and a powder rich in soluble dietary fiber (SDFP). Compared with insoluble dietary fiber, soluble dietary fiber shows higher viscosities and could decrease blood glucose and cholesterol. Moreover, the gelation capacity of soluble dietary fiber has health benefits to the consumers and allowed its use as thickeners and to produce gels and edible films.

In an embodiment, the present disclosure relates to a fractionation process to obtain different olive pomace-derived ingredients: stone-rich powder 1 and 2, liquid-rich powder and pulp-rich powder. Additionally, the pulp-rich powder could also be submitted to a chemical process to obtain a soluble dietary fiber enriched powder.

Moreover, the present disclosure proposes a fractionation strategy to achieve a maximal valorisation of whole olive pomace components to reach the "zero waste" goal.

In an embodiment, the present disclosure discloses a method for fractionating olive pomace comprising the following steps:
centrifuging olive pomace to obtain a liquid moiety and a solid moiety;
freeze-drying said liquid moiety with a cryoprotectant and milling to obtain a liquid-rich powder;
convectively drying said solid moiety;
milling and sieving said dried solid moiety to obtain a pulp-rich and/or at least one stone-rich powder from olive pomace, depending on the granulometry of the powder;
wherein the method is performed in the absence of a solvent.

In a further embodiment, the present disclosure discloses a method, wherein centrifugation is run between 5000 g and 15000 g between 5 to 15 minutes, preferably at 10000 g for around 10 minutes.

In a further embodiment, the present disclosure discloses a method, wherein the freeze-drying step is performed with mannitol, preferably at 1-3% (w/w), more preferably 2% (w/w), as a cryoprotectant to prevent aggregation.

In a further embodiment, the present disclosure discloses a method, wherein the step of convectively drying is performed at a temperature of between 50°C and 100°C, preferably at 90 °C.

In a further embodiment, the present disclosure discloses a method, wherein the step of milling is performed with a milling device, namely a coffee grinder.

In a further embodiment, the present disclosure discloses a method, wherein the step of sieving is performed using a mesh 10 (size of the mesh ranges between 1000 -2000 µm) to obtain a stone-rich powder 1; or a mesh 30 (size of the mesh ranges between 400 - 600 µm) to obtain a stone-rich powder 2, or a mesh 100 (size of the mesh ranges between 75-150 µm) to obtain the pulp-rich powder.

In a further embodiment, the present disclosure discloses a method further comprising the steps of:
submitting the pulp-rich powder to H₂O₂ at 5% (w/v);
stirring at a constant rating for between 1-2 hours;
washing twice using distilled water;
drying the solid fraction at a temperature between 50°C - 70 °C, preferably 60 °C, to obtain a blanched powder;
filtration;
freeze-drying to obtain a soluble dietary fibre powder.

In a further embodiment, the present disclosure discloses a powder comprising at least one insoluble dietary fibre, hydroxytyrosol, and unsaturated fatty acids.

In a further embodiment, the present disclosure discloses a powder according to any of the previous claims comprising mannitol, potassium and hydroxytyrosol.

In a further embodiment, the present disclosure discloses a powder comprising mannitol, soluble dietary fibers, potassium and hydroxytyrosol.

In a further embodiment, the present disclosure discloses a powder wherein the calorific potential is between 18 and 20 MJ/ kg, more preferably 19 MJ/ kg.

In a further embodiment, the present disclosure discloses a powder, preferably liquid powder from at least one of the previous embodiments, wherein the content of total phenolic compounds is between 20 and 40 mg of gallic acid equivalents per g of dried powder, preferably between 29 to 32 mg of gallic acid equivalents per g of dried powder, more preferably 30 mg of gallic acid equivalents per g of dried powder.

In a further embodiment, the present disclosure discloses a powder, preferably pulp rich powder according to at least one of the previous embodiments, wherein the content of phenolic compounds per g of dried powder is between 3 and 10 mg of gallic acid equivalents, preferably between 4 mg of gallic acid equivalents and 8 mg of gallic acid equivalents.

In a further embodiment, the present disclosure discloses a powder for use as food additive and/or food preservative.

In a further embodiment, the present disclosure discloses a powder, preferably stone-rich powder according to at least one of the previous embodiments, wherein the particle size is between 400 µm and 1000 µm.

In a further embodiment, the present disclosure discloses a powder, preferably stone-rich powder according to at least one of the previous embodiments wherein the phenolic compound amount is between 3 - 5 mg of gallic acid equivalents per gram, preferably 4 mg of gallic acid equivalents per gram.

In a further embodiment, the present disclosure discloses a powder, preferably pulp-rich powder according to at least one of the previous embodiments for use as a nutraceutical as a source of dietary fibre, as a faecal bulking agent, fermentation substrate and antioxidant compounds source.

In a further embodiment, the present disclosure discloses a powder, preferably pulp-rich powder according to at least one of the previous embodiments for use as a nutraceutical comprising monounsaturated fatty acids between 15 and 20 g per g of dry weight to promote healthy cholesterol levels.

In a further embodiment, the present disclosure discloses a pulp-rich powder according to at least one of the previous embodiments comprising dietary fibre, oleic acid, hydroxytyrosol.

In a further embodiment, the present disclosure discloses a liquid-rich powder according to at least one of the previous embodiments comprising
8 - 9 % of dietary fibre;
2 - 3 % of moisture;
0.8 - 1 % of protein;
0.7 - 1 % of lipids;
8 - 9 % of ashes;
70 - 77 % of carbohydrates;
in grams per 100 g of the dry weight of powder.

In a further embodiment, the present disclosure discloses a pulp-rich powder according to at least one of the previous embodiments comprising
60 - 62 % of dietary fibre, 51-52 % of which is insoluble dietary fibre;
3 - 4 % of moisture;
11 - 12 % of protein;
18 - 20 % of lipids;
1.5 - 2% of ashes;
2 - 3 % of carbohydrates;
in grams per 100 g of the dry weight of powder.

In an additional embodiment, the disclosure discloses the use of the powder of any of the previous embodiments as a solid biofuel.

In an additional embodiment, the disclosure discloses the use of the powder of any of the previous embodiments for production of thickeners, gels and edible films.

In an additional embodiment, the disclosure discloses the use of the powder of any of the previous embodiments for cosmetic applications, namely as an exfoliating agent for cosmetic products.

In an additional embodiment, the disclosure discloses the use of the powder of any of the previous embodiments in food industry, namely in food products to prevent cardiovascular diseases.

In an additional embodiment, the disclosure discloses the use of the powder of any of the previous embodiments as a cardiovascular preventing agent in foodstuff.

In an additional embodiment, the disclosure discloses the use of the powder of any of the previous embodiments as a food additive, food supplement, prebiotic or as a nutraceutical.

### DETAILED DISCLOSURE OF THE INVENTION

The sustainable fractionation process of olive pomace of the present disclosure relates to the production of value-added ingredients and products without any consumption of water nor chemicals, namely solvents, and with low energy consumption in drying process.

In the present disclosure, whole olive pomace is fractionated by centrifugation (10,000 xg for 10 min). In an embodiment, the liquid fraction was freeze-dried (Telstar Lyo Quest HT 40) with 2% (v/v) of mannitol (as a cryoprotectant to prevent aggregation) and the powder obtained was denominated liquid-enriched olive pomace powder (LOPP).

In an embodiment, the solid fraction (which in general represents 71% of the whole dried olive pomace) was dried using any drying process, preferably convective drying. The olive pomace solid fraction was dried at 90°C until levels of water activity (a_{w}) are below 0.4, in order to obtain a stable product. Previous experiments were performed to establish the drying temperature (50 °C, 70 °C and 90 °C). The highest temperature showed the best results regarding the total phenolic compound and antioxidant activity.

In an embodiment, the dried solid fraction was milled using a coffee grinder and sieved. After the sieving process, three different products were obtained: the fraction retained until mesh 10 (SOP 1, size of the mesh ranges between 1000- 200 µm), the fraction until mesh 30 (SOP 2) and the fraction below mesh 30 (POPP, size of the mesh ranges between 400- 600 µm).

In an embodiment, the sieving process was essential to isolate all the pieces of stones from POPP (free of physical hazards) and to obtain a better solid biofuel and exfoliating agent. The POPP represents 51.55% of the total dry weight (DW) solid fraction. The particle size of POPP ranged between 400 - 75 µm.The option by a relatively large particle size of POPP was made to maintain the hydration characteristics and the microtexture of the fiber. The average mesh most representative in POPP was the 100 mesh (size of the mesh ranges between 75-150 µm)). The particle size distribution of olive pomace solid fraction is shown in **Table 1.**

**Table 1. The particle size distribution of solid olive pomace fraction.**

| **Olive Pomace Solid Fraction** | **Sieving Mesh Size** | **Distribution (% DW)** | |
|---|---|---|---|
| **SOP 1** | Unscreened | 5.12 ± 0.30 | 26.18 |
| | 10 (1000 - 2000 µm) | 21.03 ± 0.78 | |
| **SOP 2** | 18 (600 - 1000 µm) | 14.38 ± 0.62 | 22.84 |
| | 30 (400 - 600 µm) | 8.31 ± 0.22 | |
| **POPP** | 40 (250 - 400 µm) | 9.54 ± 0.44 | 51.55 |
| | 60 (150 - 250 µm) | 12.76 ± 1.46 | |
| | 100 (75 - 150 µm) | 22.98 ± 0.52 | |
| | 200 ( < 75 µm) | 6.00 ± 0.57 | |

In an embodiment, the ingredients and products including a stone rich powder with a good calorific potential (SOP 1), other stone/peel-rich powder with good properties are to be used as exfoliating agent (SOP 2), a liquid-rich powder rich in hydroxytyrosol and potassium (LOPP) and a pulp-rich powder which are a source of antioxidant dietary fiber and oleic acid (POPP).

In a further aspect of the present invention, the POPP is also used as a source of a soluble dietary fiber powder using a blanching process.

In an embodiment, the blanched powder (BP) soluble dietary fiber powders (SDFP) were obtained using 5% H₂O₂, solid-liquid rate (w/v) as 1:15, pH 10 and reaction time as 1.5 h with constant stirring. Followed by washing twice using distilled water. The solid fraction was oven-dried (60°C) to obtain the BP. To filtrate were added 95% (v/v) ethanol (1:3) and then allowing it to stand overnight in a refrigerator. The precipitate was freeze dried to obtain the SDFP.

In a further embodiment, SOP 1 showed higher heating value (HHV) of 19 MJ/ kg. The HHV was measured by an automatic adiabatic bomb calorimeter (Parr calorimeter Type 6200) in duplicate (1g of sample) according to EN 14918. The HHV of crude olive pomace was also measured (22 MJ/ kg). The HHV of SOP 1 was lower in comparison to crude olive pomace with a small variation of 3 MJ/ kg. However, the SOP 1 exhibited better safety combustion performance (free of oil and low weight particles). The oil and low weight particle generate uncontrolled combustions and emissions, which could disturb combustion processes. Furthermore, the direct combustion of crude olive pomace also represents a wastage of bioactive compounds.

In a particular embodiment, the HHV values of SOP 1 are identical to that of hardwoods as oak wood (18.70 MJ/ kg) and pine bark (18.30 MJ/kg) (Ingram et al., 2008) and most agricultural residues [e.g. vine shoots (18.30 MJ/ kg), palm kernel (18.67 MJ/ kg)] (Vamvuka & Kakaras, 2011). The moisture content of SOP 1 showed a moisture content of 6% and a pH value of 4.3.

Additionally, the values of energy content and moisture acquired allowed to validate that SOP 1 has all the characteristics to be used as solid biofuel, namely reasonable HHV value, better and more safety combustion performance (more controlled combustion and emissions).

In another particular embodiment, the SOP 2 composed by small pieces of stones and peel exhibit a high content of crude fiber (64 %) and a considerable amount of total phenolic compounds (442 mg gallic acid equivalents (GAE)/ 100 g DW). Lipids (6 %) and protein (5%) were also identified. The particle size of 1000 - 400 µm were considered larger, which turn SOP 2 best suited for body scrub. The phenolic compounds present in this fraction allowed also to improve the oxidative stability and shelf life of cosmetic formulations.

In an embodiment, LOPP exhibit high ash, soluble sugar and organic acid content. The potassium amount in LOPP is higher (5.4%). Higher consumption of potassium is associated with the prevention of cardiovascular diseases and the maintenance of normal blood pressure. The LOPP comprises high potassium and minimal sodium content, as well as a mixture of other minerals (phosphorous, magnesium and calcium) which could mask the bitter taste of potassium. These LOPP characteristics could be explored by the food industry, not only in the mineral fortification to cardiovascular disease prevention/reduction but also as taste agent/ replacer of sodium chloride.

In an embodiment, the content of soluble sugars and organic acids is substantially high in LOPP, as a result of the liquid-solid fractionation process. The high-performance liquid chromatography (HPLC) analysis of soluble sugars and organic acids allowed the identification of mannitol and formic acid as the most representative sugar and organic acid, respectively **(Table 2).**

**Table 2. Chemical composition of olive pomace powders (g/ 100 g DW).**

| | | | ***LOPP*** | ***POPP*** |
|---|---|---|---|---|
| **Proximate Composition** | | *Moisture* | 3.37 ± 0.16^{a} | 3.76 ± 0.16 ^{b} |
| | | *Protein* | 1.12 ± 0.15 ^{a} | 10.82 ± 0.09 ^{b} |
| | | *Lipid* | 0.80 ± 0.04 ^{a} | 19.95 ± 0.70 ^{b} |
| | | *Ash* | 8.46 ± 0.54^{a} | 2.19 ± 0.02 ^{b} |
| **Minerals** | | *P* | 0.48± 0.00^{a} | 0.159 ± 0.02 ^{b} |
| | | *Mg* | 0.15 ± 0.00^{a} | 0.050 ± 0.01 ^{b} |
| | | *Ca* | 0.13 ± 0.00 ^{a} | 0.165 ± 0.02 ^{a} |
| | | *Na* | 0.02 ± 0.00 | * |
| | | *K* | 5.43 ± 0.11^{a} | 1.756 ± 0.22^{b} |
| **Sugars** | | *Glucose* | 12.57 ± 1.11 ^{a} | 3.53 ± 0.28 ^{b} |
| | | *Fructose* | 1.64 ± 0.14 ^{a} | 0.41 ± 0.06 ^{b} |
| | | *Mannitol* | 14.11 ± 1.24 ^{a} | 0.89 ± 0.05 ^{b} |
| **Organic acids** | | *Lactic* | 0.41 ± 0.02^{a} | 0.12 ± 0.00 ^{b} |
| | | *Formic* | 1.03 ± 0.07 ^{a} | 0.45 ± 0.04 ^{b} |
| | | *Acetic* | 0.26 ± 0.01 ^{a} | 0.05 ± 0.00 ^{b} |
| **Amino acids** | ***Non-essential*** | *Asp* | * | 2.21 ± 0.23 |
| | | *Glu* | * | 2.16 ± 0.26 |
| | | *Gln* | * | 0.85 ± 0.07 |
| | | *Ala* | * | 0.60 ± 0.06 |
| | | *Arg* | * | 0.57 ± 0.02 |
| | | *Cys* | * | 0.90 ± 0.08 |
| | ***Essential*** | *His* | * | 2.14 ± 0.12 |
| | | *Thr* | * | 0.16 ± 0.01 |
| | | *Tyr* | * | 0.37 ± 0.04 |
| | | *Val* | * | 0.71 ± 0.04 |
| | | *Met* | * | 0.27 ± 0.01 |
| | | *Trp* | * | 1.19 ± 0.09 |
| | | *Phe* | * | 1.10 ± 0.03 |
| **Fatty acids** | ***Saturated*** | *Myristic C14:0* | 0.03 ± 0.00 | ND |
| | | *Palmitic C16:0* | 0.12 ± 0.02 ^{a} | 3.37 ± 0.04 |
| | | *Estearic C18:0* | 0.02 ± 0.00 ^{a} | 0.50 ± 0.01 |
| | | *Archidic C20:0* | * | 0.11 ± 0.00 |
| | ***Unsaturated*** | *Palmitoleic C16:1 c9* | 0.01 ± 0.00 ^{a} | 0.38 ± 0.00 |
| | | *Oleic C18:1 c9* | 0.52 ± 0.07^{a} | 16.06 ± 0.19 |
| | | *cis-Vaccenic C18:1 c11* | 0.03 ± 0.00^{a} | 0.78 ± 0.01 |
| | | *Linoleic C18:2 c9c12* | 0.04 ± 0.01 ^{a} | 1.47 ± 0.02 |
| | | *α-linolenic α C18:3 c9c12c15* | 0.01 ± 0.00 | 0.17 ± 0.00 |

| | | | | |
|---|---|---|---|---|
| * < LOD. *Asp* - *Aspartic acid; Glu* - *Glutamic acid; Gln* - *Glutamine; Ala* - *Alanine; Arg* - *Arginine; Cys* - *Cysteine; His* - *Histidine; Thr* - *Threonine; Tyr* - *Tyrosine; Val* - *Valine; Met* - *Methionine; Trp - Tryptophan; Phe* - *Phenylalanine. Results are the means of three determinations ± standard deviation. Different letters in the same column are significantly different as determined by t-Student test (p< 0.05)* | | | | |

In an embodiment, the high mannitol content in LOPP (14.11 ± 1.24 g/ 100 g DW) could be an asset to its application as a food ingredient: (1) act as an antioxidant against oxidative damage by oxygen radicals; (2) Non-metabolizable sweetener (uptake of mannitol is independent of insulin) and (3) Food preservative (increase the food shelf life by reducing the sugar crystallisation). Formic acid is also a potent antimicrobial agent (Aquilina et al., 2014). Besides the formic acid, a considerable amount of lactic and acetic acid was found in olive pomace powders. These organic acids contribute to the sour milk or yoghurts like *flavour and* umami taste in fish sauce (Park et al., 2001), respectively.

In a particular embodiment, the mineral, sugar and organic acid composition of LOPP establish its applicability as food preservatives and as flavour enhancers/salt substitutes.

In an embodiment, the fiber content of LOPP is low (9 %), but soluble dietary fibre (SDF) profile allowed to affirm its composition as a hemicellulose-rich fraction. The main component was galactose (≈ 30%), followed by arabinose (≈ 24%) and glucose (≈ 21%). The SDF contains also phenolic compounds (14.56 ± 1.74 mg GAE/ g fiber DW) linked to them that reach the lower intestine (Table 4).

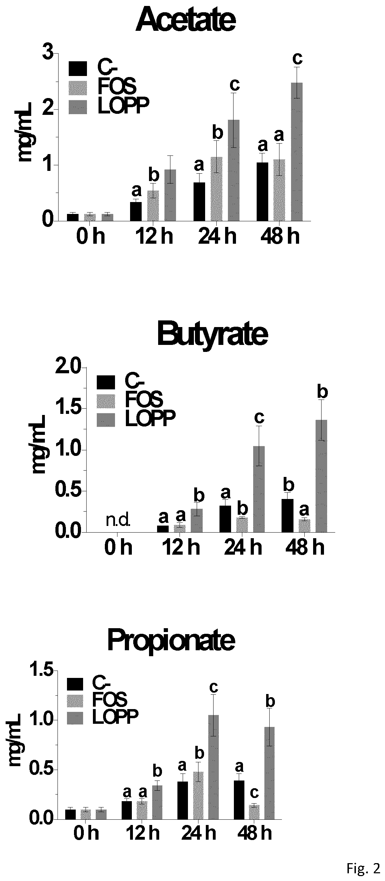

Previous studies reported a positive correlation between phenolic compounds and high galactose/arabinose content and prebiotic properties. In the *in vitro* faecal fermentation experiment performed, LOPP had a positive effect on short-chain fatty acids production (SCFA) - acetate, propionate and butyrate-than the positive control [fructooligosaccharides- (FOS)] (Fig.2). The SCFAs play important role in health and disease as modulators of cell turnover and cell differentiation in the colonic mucosa and which act also as regulators of the metabolism in systemic tissues like the liver or even the muscle (Phillips, 2013).

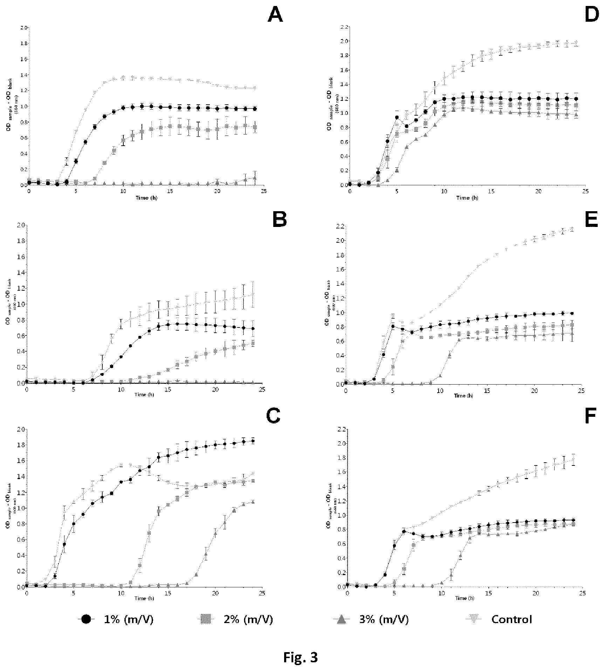

In an embodiment, additionally to the prebiotic properties, LOPP comprises a considerable antimicrobial activity and antioxidant activity. The growth of gram-positive MSSA and *Listeria monocytogenes* were completely inhibited by 3% LOPP (it can be considered as the MIC), while all other concentrations tested (i.e. sub - MIC concentrations) were capable of reducing microbial growth and caused an increase of lag phase duration. In the case of *Bacillus cereus,* the growth was completely inhibited by 16 h. Gram-positive microorganisms were more susceptible to LOPP. The growth of gram-negative microorganisms tested (*Escherichia coli, Salmonella enteritidis, Yersinia enterocolitica*) were delayed to half at 2 and 3% of LOPP and also caused a delay of the log phase (Fig. 3).

In an embodiment, regarding the antioxidant activity, LOPP showed high values using different methods - ABTS [2,2'-azino-bis(3-ethylbenzothiazoline-6-sulphonic acid)], DPPH (2,2-diphenyl-1-picrylhydrazyl) and ORAC (Oxygen Radical Absorbance Capacity)(Fig. 4).

In an embodiment, the high antimicrobial and antioxidant activity of LOPP is attributed to the presence of higher amounts of several components, namely minerals (e.g. potassium) and some organic acids such as formic, lactic and acetic acid, besides the higher concentration of polyphenols like hydroxytyrosol and its derivatives with corroborated antimicrobial and antioxidant activity. The exhibited values of antimicrobial and antioxidant activity allowed to explore LOPP not only as functional ingredients but also as preservatives.

In an embodiment, the daily amount of hydroxytyrosol and its derivatives (5 mg) that, according to the health claim approved by the EFSA (EFSA, 2011), would be needed to protect LDL particles from oxidative damage [for now only applied to olive oil (20g)] was considered in the present disclosure.

In an embodiment, due to the considerable amount of potassium and hydroxytyrosol in LOPP, the fortification with LOPP in the food industry is also within the scope of the present invention, as is the development of new food products with health benefits for use in cardiovascular prevention.

In an embodiment, LOPP exhibited a higher angiotensin-converting (ACE) inhibitory activity (91.98 ± 3.24 %) and higher α-glucosidase inhibitory activity (87.77± 1.04 %), using a tested dose (50 mg/mL). Hypertension and hyperglycaemia are commonly treated with drugs to inhibit the enzymatic activity of angiotensin-converting (ACE) and α-glucosidase, respectively. The inhibition of the angiotensin-converting enzyme (ACE) may help to reduce the formation of angiotensin II, a vasoconstrictor and ROS initiator (Alcaide-Hidalgo, Margalef, Bravo, Muguerza, & López-Huertas, 2019). On another hand, the inhibition of hydrolytic enzyme α-glucosidase may help to reduce postprandial hyperglycaemia and hence may delay the absorption of glucose.

In a further embodiment, POPP has a relatively high crude protein (11.82 %) and fat content (8.46 %). POPP revealed a higher protein content than crude olive pomace as a consequence of the concentration effect of the dry fractionation approach applied.

In an embodiment, amino acids Glu (2.16 %) and Asp (2.21 %) showed the highest values. Glu and Asp are sour stimuli and cause umami taste as the sodium salt. Asp has been also claimed with an essential role in the process of chelating minerals improving their assimilation, digestion and utilisation. The flavour enhancer and mineral fortifier potential of POPP stand out again.

Regarding fatty acids, POPP exhibits a higher level of oleic acid (corresponding to 70 % of total fatty acids) followed by linoleic acid (which corresponds to 6%), both unsaturated fatty acids with high nutritional impact.

In an embodiment, due to the high amount of unsaturated fatty acids of POPP, it is used in human nutrition because of its beneficial effects on the reduction of cholesterol and triglycerides, but also concerning the development of obesity by the restoration of the composition of gut bacteria.

In an embodiment, the nutritional quality indices of fat content [indices of thrombogenicity (TI), atherogenicity (AI) and Saturation Index (SI) were calculated to POPP. Alba *et al.* (2019) explained that AI and TI are a measure of the influence of diet on coronary heart disease. AI relates to the risk of atherosclerosis and TI values relate to the tendency to form clots in the blood vessels. Low AI and TI values were recommended. The SI indicates the relationship between the sum of saturated fatty acids (pro-thrombogenic) and unsaturated fatty acids (anti-thrombogenic). Lower SI values would be considered as healthier food (de Alba et al., 2019). POPP revealed lower AI (0.18) and TI (0.39) analogous to the values reported to olive oil (Al =0.14 and TI=0.32), and also low SI (0.20) (de Alba et al., 2019). POPP could be considered a source of healthy fat based on the nutritional quality indices.

POPP has a considerable high amount of TDF (62 %), where insoluble dietary fiber is the main component **(Table 3).**

**Table 3. Total phenolic compounds and dietary fiber composition of olive pomace powders.**

| | **Total phenolic compounds** | | **Dietary Fiber** | | |
|---|---|---|---|---|---|
| | **mg GAE/ g DW** | | **g/ 100 g DW** | | |
| | ***FPC*** | ***BPC*** | ***TDF*** | ***IDF*** | ***SDF*** |
| ***LOPP*** | 30.49 ± 1.42 ^{a} | 4.97 ± 0.36 ^{a} | 9.20 ± 1.18 ^{a} | ND | 9.20 ± 1.18 ^{a} |
| ***POPP*** | 4.48 ± 0.23 ^{b} | 8.48 ± 0.39 ^{b} | 62.06 ± 0.54 | 52.17 ± 0.01 | 9.89 ± 0.54 ^{a} |

*FPC: Free phenolic compounds extract; BPC: Bound phenolic compounds extract; TDF- Total dietary fiber; IDF* - *insoluble dietary fiber; SDF* - *Soluble dietary fiber. Results are the means of three determinations ± standard deviation. Different letters in the same column are significantly different as determined by the t-Student test (p< 0.05*).

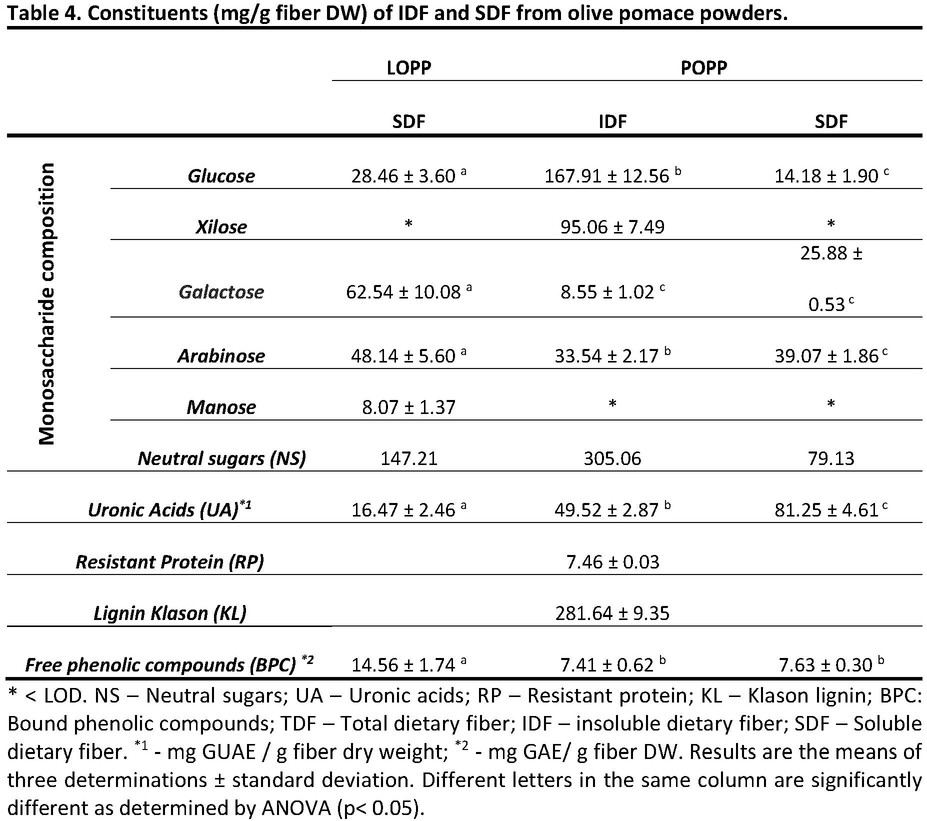

In one embodiment. POPP is considered to be an excellent source of fiber and might be of interest for the food industries like confectionery and bakery to obtain high-fiber products. The TDF content of POPP was higher than the detected in cereals, such as wheat bran (44%) and oat bran fiber (24%), generally applied in fiber-fortification of foods. Food products can be considered a "source of fiber" and labelled as such when the product contains at least 1.5 g of fiber per 100 kcal, while products containing 3 g of fiber per 100 kcal can be classified as a "high fiber food". Food products could be formulated to fulfil the condition of being a "source of fiber" or "high fiber food" using 2-4% of POPP.

Furthermore, POPP could be considered a source of antioxidant dietary fiber, with over 50% of fiber DW and high antioxidant activity (Fig.4). The antioxidant activity of POPP fiber fractions corresponded to more than half (50-60%) of the total AOXA of BPC.

In an embodiment, the monosaccharides composition reveal that insoluble dietary fibre (IDF) fraction of POPP is mostly a source of insoluble arabinoxylans and cellulose. Uronic acids were also detected in a considerable amount (19% of the total insoluble dietary fiber monosaccharides composition). Still, the main component of IDF was Klason lignin (≈ 51% of total insoluble dietary fiber composition). The complex structure of lignin has a positive effect on the protection of phenolic compounds along the gastrointestinal tract. The phenolics linked to lignin could reach the colon and play an essential role in health due to their antioxidant properties.

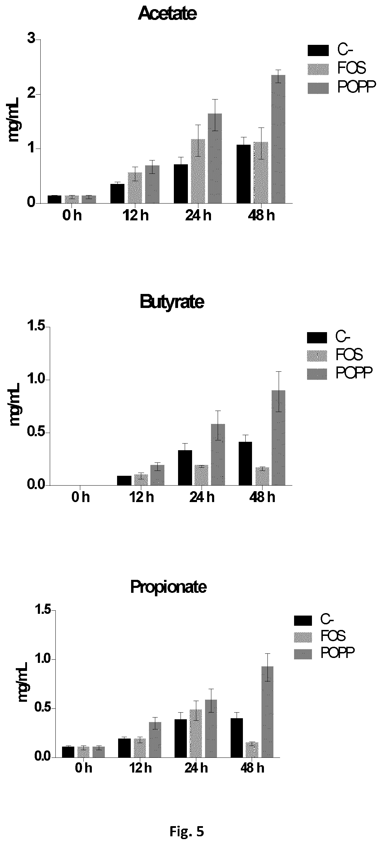

In an embodiment, the *in vitro* faecal fermentation experiment performed POPP significantly enhanced SCFA production (acetate> butyrate>propionate) **(****Fig. 5****).**

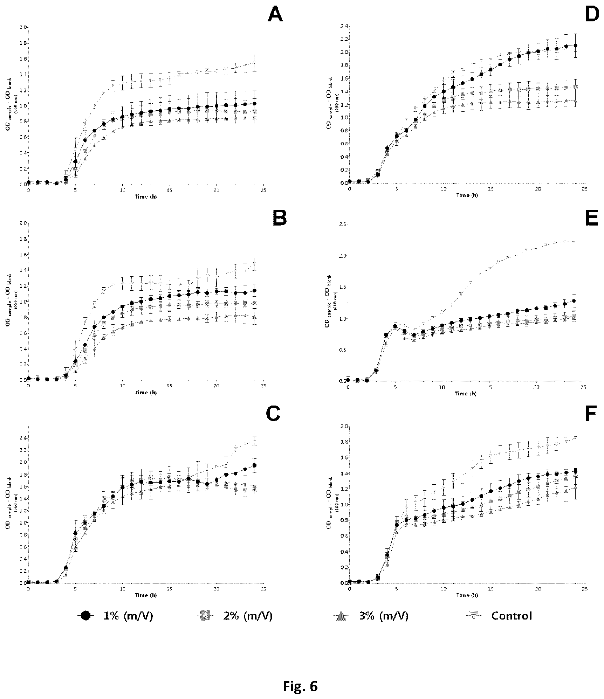

Furthermore, POPP at 2 e 3 % induced a reduction of the growth and delay the log phase of MSSA, *Listeria monocytogenes, Escherichia coli, Salmonella enteritidis* and *Yersinia enterocolitica* **(****Fig. 6****).**

In an embodiment, POPP as source of antioxidant dietary and monounsaturated fatty acids is used to develop new foods with health benefits to the gut and as fat replacers and lipid oxidation in meat products or others resembling them. The simultaneous richness in polyphenols and oleic acid of POPP offers an opportunity to obtain food products more stable to lipid peroxidation.

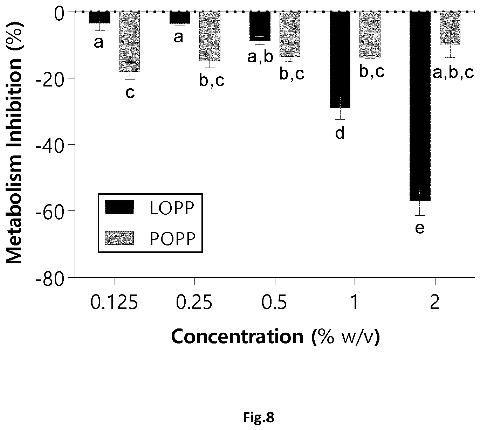

In an embodiment, the cytotoxicity of LOPP and POPP powders were studied. The LOPP and POPP were not toxic and, in an embodiment, promoted Caco-2 cells metabolism at the assayed concentration range **(****Fig.8****).**

In an embodiment, the LOPP and POPP powders meet the requirements of the food industry, namely cheap, environmentally friendly and natural, and with excellent antioxidant properties.

In an embodiment, LOPP and POPP are applied in several food matrixes, like meat products as fat and fat replacers, but also as antioxidants and dietary fibre fortifiers in for example dairy products.

In an embodiment, LOPP and POPP incorporation were tested into yoghurt as functional ingredients. Yoghurt is highly appreciated for its nutritional value and positive bioactive effects (Helal & Tagliazucchi, 2018; Oliveira & Pintado, 2015). Its health benefits are fundamentally associated with the presence of bioactive peptides and probiotics. Nevertheless, yoghurt does not contain natural fibre nor phenolic compounds. Food products can be considered a "source of fibre" and labelled as such when the product contains at least 1.5 g of fibre per 100 kcal, while products containing 3 g of fibre per 100 kcal can be classified as a "high fibre food".

In an embodiment, the incorporation of OP powders into yoghurts showed that fortification of 2% POPP fulfilled the condition on being a "source of fibre" (Table 5). The addition of 1% of LOPP to yoghurt provides the 5 mg of hydroxytyrosol and derivatives in a regular yoghurt (120 g) to obtain the health benefits to protect LDL particles from oxidative damage (Table 6). OP powder-fortified yogurt exhibited higher total phenolic content (p < 0.05) and higher radical scavenging activity (p < 0.05) compared to control yogurt (Table 6).

**Table 5. Dietary fibre fractions of OP powders (AOPP and AFOPP) and fortified yoghurts (Y-AOPP and Y-AFOPP).**

| | ***Y-LOPP*** (g/ 100 g DW) | ***Y-LOPP*** (g/ 100 g WW) | ***Y-LOPP** (g*/*100 kcal)* '*1* | ***Y-POPP*** (g/ 100 g DW) | ***Y-POPP*** (g/ 100 g WW) **1* | ***Y-POPP** (g*/*100 kcal) *1* |
|---|---|---|---|---|---|---|
| ***TDF*** | 3.62 ± 0.26 | 0.61 ± 0.04 | 1.01 ± 0.07 | 6.42 ± 0.03 | 1.07 ± 0.01 | 1.76 ± 0.01 |
| ***IDF*** | 2.24 ± 0.07 | 0.38 ± 0.01 | 0.63 ± 0.02 | 5.11 ± 0.12 | 0.85 ± 0.02 | 1.40 ± 0.03 |
| ***SDF*** | 1.38 ± 0.20 | 0.23 ± 0.03 | 0.38 ± 0.05 | 1.31 ± 0.09 | 0.22 ± 0.01 | 0.36 ± 0.02 |

| | | | | | | |
|---|---|---|---|---|---|---|
| *1 An yoghurt plain - whole milk contains 61 kcal. Results are the means of three determinations ± standard deviation. Y-LOPP - LOPP - fortified yoghurt; Y-POPP - POPP - fortified yoghurt TDF-Total dietary fiber; IDF - insoluble dietary fiber; SDF - Soluble dietary fiber; WW - Wet weight. Results are the means of three determinations ± standard deviation. | | | | | | |

**Table 6. Total phenolic compounds content and antioxidant activity with ABTS and DPPH of fortified yoghurts (Y-AOPP and Y-AFOPP) and control (Y-control).**

| | **Y-Control** | **Y - LOPP** | **Y-POPP** |
|---|---|---|---|
| *Hydroxytyrosol* (mg/100 g DW) | ND | 12.46 ± 0.95 | 0.26 ± 0.06 |
| *Hydroxytyrosol derivatives* (mg/100 g DW) | ND | 3.12 ± 0.03 | 0.91 ±0.05 |
| *Protocatechuic acid* (mg/100 g DW) | ND | ND | 1.22 ± 0.05 |
| *Tyrosol* (mg/100 g DW) | ND | 3.72 ± 0.13 | 0.20 ± 0.02 |
| *Tyrosol derivatives* (mg/100 g DW) | ND | 4.90 ± 0.29 | 0.16 ± 0.01 |
| *Caffeic acid* (mg/100 g DW) | ND | 0.48 ± 0.04 | 0.37 ± 0.09 |
| *p-Coumaric acid* (mg/100 g DW) | ND | 0.31 ± 0.03 | 0.32 ± 0.08 |
| *Luteolin* (mg/100 g DW) | ND | ND | 1.51 ± 0.70 |
| ***Total phenolic compounds** (mg GAE*/*g DW)* | 0.44 ± 0.12^{a} | 1.79 ± 0.17 | 1.15 ± 0.09 |
| ***ABTS*** (mM TE/g) | 0.92 ± 0.08 ^{a} | 7.24 ± 0.34 | 4.11 ± 0.21 |

ND - not detected; GAE - gallic acid equivalents; TE - Trolox equivalents. Results are the means of three determinations ± standard deviation. Different letters in the same column are significantly different as determined by two-way ANOVA and analysed by Tukey's post hoc test (p< 0.05).

In an embodiment, the BP exhibit a higher content of fiber (72.07 ± 0.93 %) and a better ratio of SDF/IDF (0.23) than POPP (0.19). The SDFP exhibit high soluble dietary fiber content (40.31 ± 2.45 %), which could be explored to food industry as thickeners and to produce gels and edible films (Table 7).

**Table 7. Total phenolic compounds and dietary fiber composition of olive pomace powders.**

| | **Dietary Fiber (g/ 100 g DW)** | | |
|---|---|---|---|
| | ***TDF*** | ***IDF*** | ***SDF*** |
| ***BP*** | 72.07 ± 0.93 ^{a} | 58.62 ± 1.46 | 13.45 ± 1.27 ^{a} |
| ***SDFP*** | 42.84 ± 2.23 | 2.53 ± 0.22 | 40.31 ± 2.45 ^{a} |

TDF - Total dietary fiber; IDF - insoluble dietary fiber; SDF - Soluble dietary fiber. Results are the means of three determinations ± standard deviation. Different letters in the same column are significantly different as determined by the t-Student test (p< 0.05).

This invention brings new insight to develop a sustainable solution for wastes resulting from the olive oil production, with capacity to added value and a commitment to full valorization waste.

The term "comprising" whenever used in this document is intended to indicate the presence of stated features, integers, steps, components, but not to preclude the presence or addition of one or more other features, integers, steps, components or groups thereof.

The disclosure is of course not in any way restricted to the embodiments described and a person with ordinary skill in the art will foresee many possibilities to modifications thereof without departing from the basic idea of the disclosure as defined in the claims.

The above-described embodiments are obviously combinable.

### References

[1] EFSA Journal 2010;8(10):1735 - https://efsa.onlinelibrary.wiley.com/doi/pdf/10.2903/j.efsa.2010.1735 -
[2] EFSA Journal 2011;9(4):2043 - https://efsa.onlinelibrary.wiley.com/doi/epdf/10.2903/j.efsa.2011.2043
[3] EFSA Journal 2016;14(10):4592 - https://www.efsa.europa.eu/en/efsajournal/pub/4592
[4] EFSA Journal 2011;9(4):2033 - https://efsa.onlinelibrary.wiley.com/doi/pdf/10.2903/j.efsa.2011.2033

## Claims

1. Method for fractionating olive pomace comprising the following steps:
centrifuging olive pomace to obtain a liquid moiety and a solid moiety;
freeze-drying said liquid moiety with a cryoprotectant and milling to obtain a liquid-rich powder;
convectively drying said solid moiety;
milling and sieving said dried solid moiety to obtain a pulp-rich and/or at least one stone-rich powder from olive pomace, depending on the granulometry of the powder;
wherein the method is performed in the absence of a solvent,
wherein centrifugation is run between 5000 g and 15000 g between 5 to 15 minutes,
wherein the freeze-drying step is performed with 1-3% (w/w) mannitol as a cryoprotectant to prevent aggregation.

2. Method according to any of the previous claims, wherein centrifugation is run between 5000 g and 10000 g between 5 to 15 minutes, preferably around 10 minutes.

3. Method according to any of the previous claims, wherein the freeze-drying step is performed with 2 -3 % (w/w) mannitol, as a cryoprotectant to prevent aggregation.

4. Method according to any of the previous claims, wherein the step of sieving is performed using a mesh size between 1000 - 2000 µm to obtain a stone-rich powder 1; or a mesh size between 400 - 600 µm to obtain a stone-rich powder 2, or a mesh size between 75 - 150 µm to obtain the pulp-rich powder.

5. Powder obtained from the method according to any of the previous claims comprising at least one insoluble dietary fibre, hydroxytyrosol, and unsaturated fatty acids, preferably wherein the particle size is between 400 µm and 1000 µm.

6. Powder according to any of the previous claims comprising mannitol, potassium and hydroxytyrosol, preferably comprising mannitol, soluble dietary fibers, potassium and hydroxytyrosol.

7. Powder according to any of the previous claims wherein the calorific potential is between 18 and 20 MJ/ kg, more preferably 19 MJ/ kg.

8. Powder, preferably liquid rich powder according to any of the previous claims, wherein the content of total phenolic compounds is between 20 and 40 mg of gallic acid equivalents per g of dried powder, preferably between 29 to 32 mg of gallic acid equivalents per g of dried powder, more preferably 30 mg of gallic acid equivalents per g of dried powder.

9. Powder, preferably stone-rich powder according to any of the previous claims wherein the phenolic compound amount is between 3 - 5 mg of gallic acid equivalents per gram, preferably 4 mg of gallic acid equivalents per gram.

10. Powder, preferably pulp-rich powder according to any of the previous claims for use as a nutraceutical as a source of dietary fibre, as a faecal bulking agent, fermentation substrate and antioxidant compounds source; preferably use as a nutraceutical comprising monounsaturated fatty acids between 15 and 20 g per g of dry weight to promote healthy cholesterol levels.

11. Powder, preferably pulp-rich powder according to any of the previous claims for use as a nutraceutical comprising monounsaturated fatty acids between 15 and 20 g per g of dry weight to promote healthy cholesterol levels, preferably comprising dietary fibre, oleic acid, hydroxytyrosol.

12. Liquid-rich powder according to any of the previous claims comprising
8 - 9 % (wt/wt) of dietary fibre; preferably 60 - 62 % (wt/wt) of dietary fibre, 51-52 % (wt/wt) of which is insoluble dietary fibre;
2 - 3 % (wt/wt) of moisture, 3 - 4 % (wt/wt) of moisture;0.8 - 1 % (wt/wt) of protein, preferably 11 - 12 % (wt/wt) of protein;
0.7 - 1 % (wt/wt) of lipids, preferably 18 - 20 % (wt/wt) of lipids;
8 - 9 % (wt/wt) of ashes, preferably 1.5 - 2% 1.5 - 2% of ashes of ashes;
70 - 77 % (wt/wt) of carbohydrates, preferably 2 - 3 % of carbohydrates; in grams per 100 g of the dry weight of powder.

13. Use of the powder described in any of the previous claims as a solid biofuel or for cosmetic applications, namely as an exfoliating agent for cosmetic products.

14. Use of the powder described in any of the previous claims in food industry, namely in food products to prevent cardiovascular diseases.

15. Use of the powder described in any of the previous claims as a cardiovascular preventing agent in a foodstuff, preferably a food additive, food supplement, prebiotic or as a nutraceutical, more preferably as thickeners, gel or edible film.
